# EUROPEAN PATENT SPECIFICATION

(11) **EP 4 186 971 B1**
(45) Date of publication and mention of the grant of the patent: **06.03.2024**
(21) Application number: 22075013.7
(22) Date of filing: 23.11.2022
(51) Int. Cl.: C12M 1/32, B01F 31/24, B01F 35/42, C12M 1/00, C12M 3/06

(54) **A METHOD OF INCUBATING CELLS IN A CELL-GROWTH UNIT CLAMPED TO A BASE PLATE OF A SHAKER DEVICE, AND A CLAMP**
VERFAHREN ZUR INKUBATION VON ZELLEN IN EINER AN EINER GRUNDPLATTE EINER SCHÜTTLERVORRICHTUNG GEKLEMMTEN ZELLWACHSTUMSEINHEIT UND KLEMME
PROCÉDÉ D'INCUBATION DE CELLULES DANS UNE UNITÉ DE CROISSANCE CELLULAIRE SERRÉE SUR UNE PLAQUE DE BASE D'UN DISPOSITIF SECOUEUR, ET PINCE

(30) Priority: 26.11.2021 NL 1044220
(43) Date of publication of application: 31.05.2023
(73) Proprietor: Enzyscreen B.V., 2105 MB Heemstede (NL)
(72) Inventor: Duetz, Wouter Adriaan, 2105 MB Heemstede (NL); Joordens, Servaes, 2105 MB Heemstede (NL)
(74) Representative: de Hoog, Johannes Hendrik

(56) References cited:
- US-A1- 2005 287 573
- US-A1- 2012 237 416
- US-A1- 2017 312 709
- US-A1- 2018 001 285

## Description

The invention concerns a method of incubating cells in a cell-growth unit (such as a microtiter plate), wherein the cell-growth unit is placed on a moveable base plate of a shaker device, said shaker device comprising a clamp, said clamp comprising a frame, wherein the method of incubating the cells comprises the steps of
- clamping of the cell-growth unit to the base plate,
- performing the incubation of the cells, and
- releasing the clamp to release the cell-growth unit.

In the biotechnological field, cells are incubated to perform experiments or to grow them. To that end, use has been made of shakers for a long time. The cells are incubated in a removable cell-growth unit that is releasably fixed to the base plate while being moved. The cell-growth unit comprises one or typically more lumen containing a medium for the cells, such as a growth medium or containing one ore more reagents. To prevent the cell-growth unit from moving when the shaker is in operation, the cell-growth unit is pressed against the base plate using the clamp. In more recent times, lab technicians are helped by robots comprising a robot arm for routine manipulation. Obviously cost and also safety are concerns, so the robots will typically be simple, light and not very strong.

The goal of the invention is to provide a method that allows such a robot to clamp down a cell-growth unit reliably to the base plate and, after incubating the cells, to easily release the clamp to remove said cell-growth unit from the base plate.

The goal is achieved using a shaker device, wherein
- the clamp is at a first end of said frame attached to the base plate, wherein the clamp is rotatable about a first axis of rotation parallel to the base plate, and at a second end of the frame opposite of the first end the frame is provided with a first oblong ferromagnetic element, wherein the frame is rotatable about the first axis from a first position in which the frame is in an upright orientation to a second position in which the second end of the frame is relatively close to the base plate,
- the base plate is provided with a second oblong ferromagnetic element,

with at least one of the first and the second ferromagnetic element being a magnet,
wherein in the second position the first and second ferromagnetic element are relatively rotatable about a second axis of rotation from a first orientation in which the oblong first and second ferromagnetic elements are transverse to each other and are relatively not magnetically attracted to a second orientation in which said elements are aligned in their longitudinal directions and are relatively magnetically attracted, wherein the relative movement of one element chosen from the first and second ferromagnetic element from the first orientation to the second orientation defines a first plane and in the second orientation the longitudinal direction of the other element of the first and second ferromagnetic element is in a second plane parallel to said first plane,
wherein the step of clamping the cell-growth unit to the base plate comprises
   - placing the cell-growth unit between the location where the first end of the frame is attached to the base plate and the location of the second oblong ferromagnetic element,
   - moving the frame from the first position to the second position, and
   - with the frame in the second position relatively moving the elements to the second orientation to sandwich the cell-growth unit between the frame and the base plate, and
wherein the step of releasing the clamp comprises relatively moving the-elements towards the first orientation and then moving the frame to the first position.

Thus a method is provided wherein even a simple and relatively weak robot arm may be used to reliably clamp and release the cell-growth unit. The frame acts as a lever arm, as a result of which relatively weak magnet(s) can be used as the ferromagnetic elements, making it easier for a relatively weak robot to release the clamp.

With the method according to the invention, a human operator is still able to perform the operations manually.

Typically the first oblong ferromagnetic element will be rotated and the second oblong ferromagnetic element will be rigidly connected to the base plate.

In this application, the term cell refers to any prokaryotic or eukaryotic cell, such as bacterial cells, mammalian cells etc.

A cell-growth unit is a unit that comprises unit body, said unit body comprising one or more lumen or wells for growing the cells. Typically the unit comprises a plurality of wells, said wells being arranged in a two-dimensional array. A well-known example of such a cell-growth unit is a microtiter plate.

A very important application of the invention is one where the cell-growth unit comprises a lid for the unit body, the method allowing for securing not only the unit body but also the lid while the cell-growth unit is being moved using the shaker device during incubation. A lid is typically used to prevent ingress of unwanted contaminants, including foreign micro-organisms . The lid may comprise a membrane to allow the passage of air. A lid is typically used to avoid cross-contamination between wells, in which case the lid will comprise a resilient liner (typically made of rubber or silicone) facing the unit body. It is in particular when using such a lid that a large clamping force is required, exacerbating in particular the problem of releasing a clamp, and where the present invention is effective.

A shaker device is a shaker as known in the art for growing cells, such as a linear shaker or more typically an orbital shaker, such as disclosed in US2017/0312709 (Duetz).

According to a preferred embodiment, the frame clamps the growth-unit to the base plate via a protruding element facing the base plate in the second position at a location between 35-65% of the distance between a first end of the growth-unit closest to the first end of the frame and a second end of the growth-unit opposite of the first end of the growth unit.

This allows for uniform and secure clamping of the cell-growth unit making efficient use of the lever effect to release the cell-growth unit while clamped magnetically. The location is preferably between 40-60%, more preferably between 45-55%.

By way of example, the frame itself may locally protrude or the frame may comprise a frame body provided with an element, such as a nylon block facing the base plate in the second position.

According to a preferred embodiment, both the first and the second ferromagnetic element are magnets.

This allows for improved automatic alignment of the oblong elements in the second orientation, which makes the clamping step easy to perform using a simple robot.

According to an advantageous embodiment the first oblong ferromagnetic element is rotatably mounted to the frame.

This provides for more easy access for the robot arm and/or a human hand to release the clamp. Typically the second oblong ferromagnetic element is rigidly connected to the base plate. Typically frame will be provided with a handle for rotating the first oblong ferromagnetic element.

In case of oblong magnets with their poles at the end, it is advantageous that the movement of the frame from the first position to the second position end is performed such that in the second position the oblong magnets have their magnetic fields parallel and in opposite directions. Thus the magnets will repel each other and the landing of the frame will be relatively soft, allowing it to be performed by a robot relatively easy.

In case of magnets with the poles transverse to the second plan, it is preferred that the movement of the frame from the first position to the second position end is performed such that in the second position the oblong magnets are in the first orientation (i.e. the magnets are at a right angle with respect to each other). This renders the landing of the frame relatively soft compared to landing with the oblong magnets already aligned parallel and attracting.

In the latter case, it is preferred if the frame is arranged for biasing the oblong magnet to the first orientation if the magnet is in an intermediate orientation relatively far from the second orientation and relatively close to the first orientation. This may, for example, conveniently be achieved using a further ferromagnetic element such as a magnet or a spring. This facilitates operation by a robot, which may be quite simple. It may also help to ensure the precise orientation of the handle, making it easier for a robot arm to engage it.

According to a preferred embodiment the first oblong ferromagnetic element is mounted to the frame using a threaded axle.

This allows the clamping pressure to be adjusted.

In case of oblong magnets with their poles at the end, adjustments require one full turn or a multiple thereof. In case of magnets with the poles transverse to the second plane the adjustment can be more precise, requiring rotation over 180° or a multiple thereof.

Both of these adjustments are stepwise. However, according to a preferred embodiment the threaded axle is an axle that is strictly axially adjustable with respect to the frame. This allows for a continuous adjustment. The threaded axle will be fixed relative to the frame once the adjustment has been made, for example by clamping the threaded axle to the frame using nuts. In the present application, the term strictly axially adjustable means that the distance of the first element to the frame can be varied without changing its orientation. This is easily achieved by inserting the threaded axle in an oversized through hole in the frame.

According to a preferred embodiment rotating the first ferromagnetic element from the second to the first orientation corresponds to an axial movement of the first ferromagnetic element of the second plane towards the first plane.

This reduces the force necessary for releasing the clamp.

This may be achieved by mounting the first oblong element to a left-handed threaded rod (with the longitudinal direction of the first oblong element transverse to the centerline of the threaded rod), said rod being rotatably mounted at the second end of the frame.

According to a preferred embodiment, the base plate is provided with a barrier that blocks the movement of the frame towards the second ferromagnetic element if the first ferromagnetic element is in the second orientation and allows the rotation from the first orientation to the second orientation of the first ferromagnetic element when said first ferromagnetic element has passed said barrier.

In other words, the barrier is present
- spaced apart from the second ferromagnetic element and
- in a volume of rotation that is defined by the rotation of the first element from the first
position to the second position when the first ferromagnetic element is in the second orientation but the barrier is not present in the volume of rotation that is defined by the rotation of the first element from the first position to the second position when the first ferromagnetic element is in the first orientation.

It is preferred that the barrier comprises a bevel for biasing the first oblong ferromagnetic element towards the first orientation if said first oblong ferromagnetic element is between the second orientation and the first orientation.

According to a preferred embodiment, at least one of the base plate and the second end of the frame is provided with a stop ensuring an air gap between the oblong ferromagnetic elements when in the second orientation and second position.

By providing a gap between the magnets, the friction that would occur when relatively moving the elements from the second orientation towards the first orientation is greatly reduced, making it easy for a relatively weak robot arm to perform the release operation.

The air gap is typically less than 1 mm and preferably in the order of 0.2 mm or less. Narrower gaps mean stronger magnetic attraction, and thus allow for the use of a weaker (cheaper) magnet. The stop is preferably a height-adjustable stop, allowing minimization of the distance between the magnets in the second orientation

According to a preferred embodiment, the length to width ratios of the oblong ferromagnetic elements are independently chosen to be at least 3, and preferably at least 4. Thus the overlap in the second orientation can be more effectively reduced.

According to a preferred embodiment, the length to width ratios of the oblong ferromagnetic elements are independently chosen to be at most 10, and preferably at most 8. Thus the torque required to relatively move the magnets is effectively limited.

According to a preferred embodiment, the length to thickness ratio of the oblong ferromagnetic elements is preferably at least 10, and more preferably at least 14.

This has been found to effectively limit the torque required to relatively move the magnets.

Finally the invention relates to a clamp for clamping a cell-growth unit to a base plate of a shaker device, said clamp comprising a frame, said frame
- having a first end for attachment to the base plate, wherein the clamp is rotatable about a first axis of rotation parallel to the base plate, and
- at a second end of the frame opposite of the first end the frame being provided with a first oblong ferromagnetic element, wherein the frame is rotatable about the first axis from a first position in which the frame is in an upright orientation to a second position in which the second end of the frame is relatively close to the base plate,

wherein the clamp comprises a second oblong ferromagnetic element for attachment to the base plate,
with at least one of the first and the second ferromagnetic element being a magnet,
wherein in the second position the first and second ferromagnetic element are relatively rotatable about a second axis of rotation from a first orientation in which the oblong first and second ferromagnetic elements are transverse to each other and are relatively not magnetically attracted to a second orientation in which said elements are aligned in their longitudinal directions and are relatively magnetically attracted, wherein the relative movement of one element chosen from the first and second ferromagnetic element from the first orientation to the second orientation defines a first plane and in the second orientation the longitudinal direction of the other element of the first and second ferromagnetic element is in a second plane parallel to said first plane.

Such a clamp is suitable in the method according to the invention. The invention also comprises embodiments of the clamp with the features discussed in any of the dependent claims or discussion thereof in any combination.

The invention is now exemplified with reference to the drawings.
Fig. 1 shows a shaker provided with growth-units and clamps.
Fig. 2A to Fig. 2E show a detail of the shaker of Fig. 1 and illustrate the stages of clamping a growth-unit to the shaker.
Fig. 3 corresponds to the view of Fig. 2D, illustrating further aspects of the clamp..
Fig. 4 corresponds to Fig. 2C showing a preferred embodiment of the clamp.

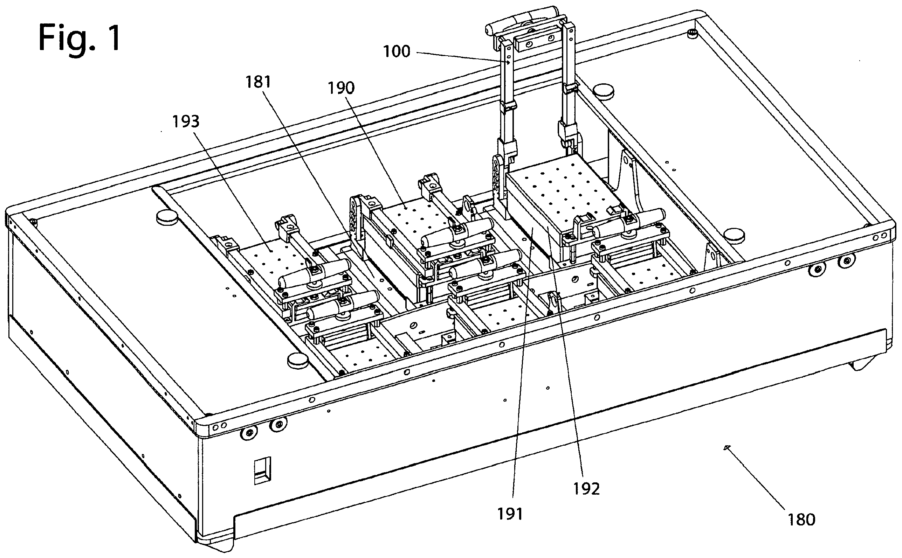

Fig. 1 shows a shaker 180 provided with growth-units 190, here six. The shaker comprises a base plate 181 provided with clamps 100, also six. Clamp 100' is in a first upright position, whereas the other clamps are in a second, clamping position.

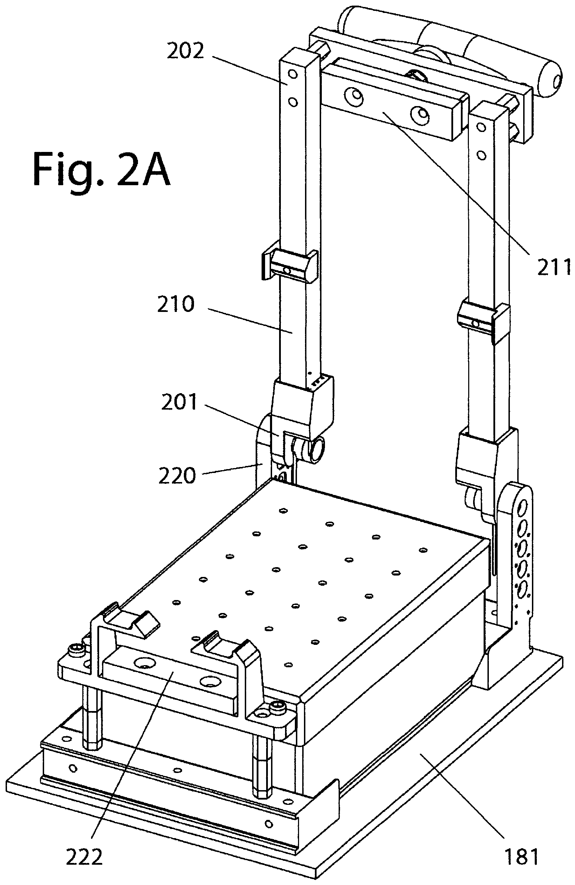

In the embodiment discussed here, the growth-units 190 comprise a growth-unit body 191 and are provided with a lid 192. This lid 192 is to be held tightly against the growth-unit body 191 while the contents of the growth-unit 190 are shaken using the shaker 180. The lid 192 comprises holes 193 allowing the passage of air to cells in the growth-unit body 191. To avoid contamination, a membrane is provided at the inside of the lid 192. The lid 192 also contains a resilient inlay (with corresponding holes) to help to prevent cross-contamination between lumen of the growth-unit body 192. This is all state of the art and doesn't require further elucidation. Fig. 2A shows the clamp 100 in the first upright position relative to the base plate 181. The clamp 100 comprises a frame 210 that at a first end 201 of the clamp 100 is rotatably connected to the base plate 181 via an upright 220. The (first) axis of rotation is parallel to the base plate 181. At a second end 202 of the clamp 100, the frame 210 is provided with a first oblong ferromagnetic element 211 rigidly connected to a threaded rod 288 that is rotabable about a second axis of rotation transverse to the plane of the frame 210. A handle 212 is provided for rotating the first element 211, which may be done manually or using a robot arm (not shown), from a first orientation to a second orientation and back.

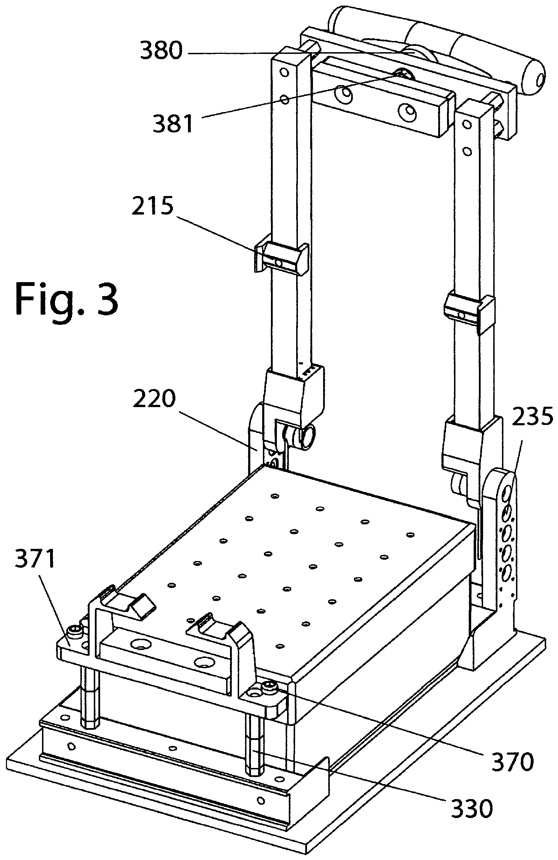

The base plate 181 is provided with a second oblong ferromagnetic element 222 (a magnet), as discussed in more detail with reference to Fig. 3.

In the example discussed here, the magnets were Neodynium N35 magnets (50 x 10 x 3 mm) with the poles at the opposite main planes.

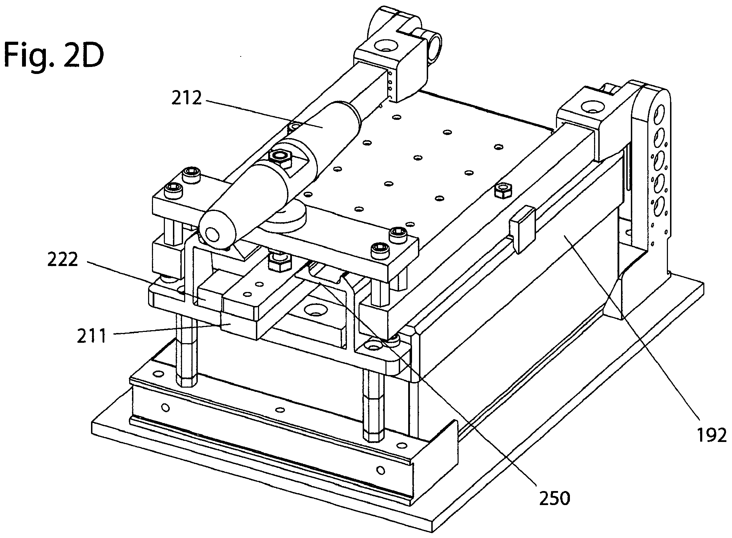

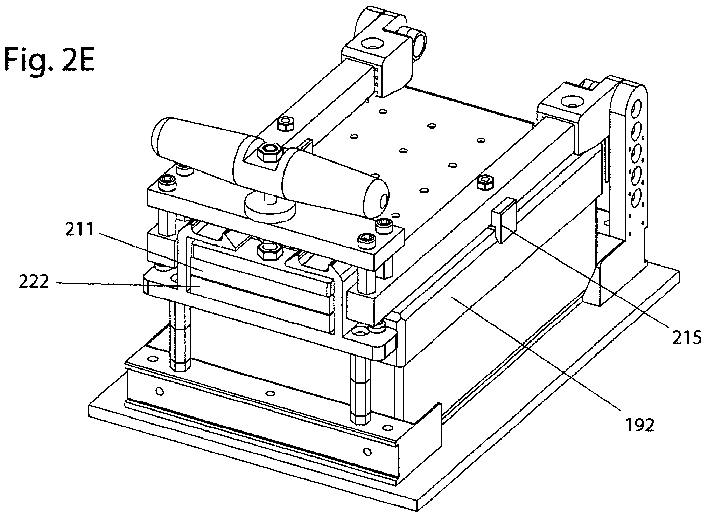

If the frame 210 is in the second (horizontal) position, the elements 211, 222 are transverse (Fig. 2D) to each other (relatively not attracting) in the first orientation whereas in the second orientation they are parallel and relatively attracting (Fig. 2E).

In the embodiment discussed here, barriers 250 are provided that serve to protect the first element 211 from colliding with the second element 222. A passage 255 is provided allowing the first element 211 to pass to the second position if said first element 211 is in the first orientation. To clamp the growth-unit 190 to the base plate 181 of the shaker 180, the frame 210 is rotated about the first axis of rotation towards the second position.

If the first element 211 is not yet in an orientation that allows it to pass through the passage 255, it is rotated about the second axis of rotation to the first orientation. As a human operator may be more sloppy than a robot to sufficiently rotate the first element 211, the barriers 250 are provided at distal ends thereof with bevels 251, biasing the first element 211 towards the first orientation allowing passage.

Once the first element 211 clears the passage 255, it is rotated to the second orientation where the magnets 211, 222 engage strongly and the magnets will automatically align to the second orientation. This causes protruding elements 215 (here nylon blocks) on the frame 210 to clamp the growth-unit 190 strongly to the base plate 181, thanks to the lever action exerted. In the embodiment discussed here, the two magnets 211, 222 require a force of 50 N to separate them in the direction of the movement from the second position to the first position, and with the protruding elements 215 halfway between the first axis of rotation and the first magnet 211 a force of about 100 N is exerted.

After incubating cells in the growth-unit 190, the above procedure is reversed (from Fig. 2E to Fig. 2A) to release the growth-unit 190. The torque required to rotate the first magnet 211 is 0.4 Nm and the force to separate the magnets 211, 222 in that first orientation was less than 1 Newton.

In the above example, the Ratio of the Length to Width was 5. For a shorter magnet (Ratio = 4), the force to separate the magnets was larger, whereas for a longer magnet (Ratio = 6) the required torque was twice that of the above 50 mm magnets.

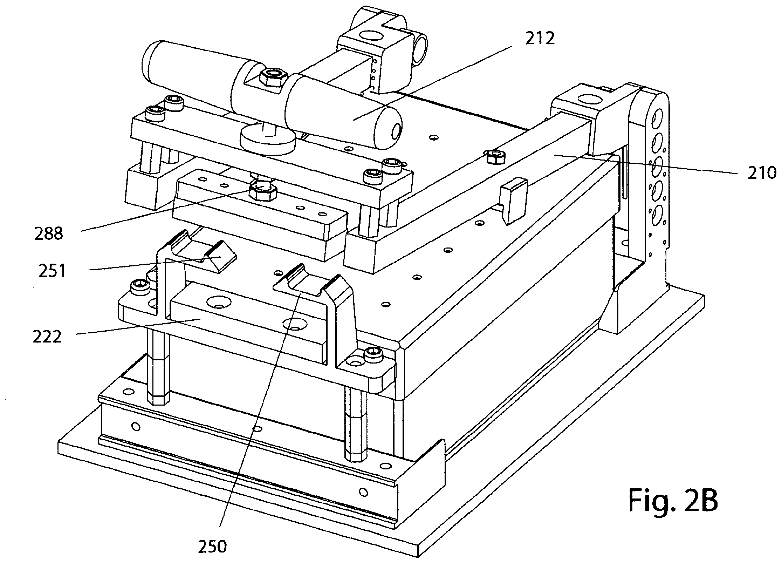

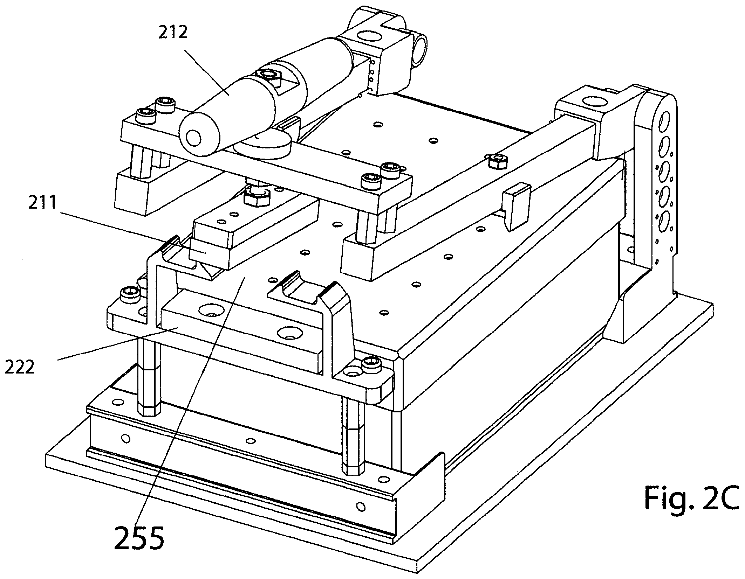

Fig. 3 is identical to Fig. 2 and is used to illustrate further advantageous aspects of the embodiment discussed above.

To handle growth-units of different heights, the rotatable frame 210 can be attached at a selection of heights to which end the upright 220 is provided with holes 325.

The second magnet 222 is attached to the base plate 181 at a corresponding height by use of spacers 330.

The base plate 181 is provided with stops 370 (adjustable bolts 370) mounted on a cross-beam 371 carrying the second magnet 222, ensuring an air gap between the oblong ferromagnetic elements 211, 222 when in the second position and second orientation. This reduces the friction and hence the torque required to move the first magnet 211 to the first orientation.

A knurled nut 380 may be used for one or two different purposes. It may be used to determine the maximum force exerted by the protruding elements 215 on the lid 192 (which force is ultimately limited by the strength of the magnets 211, 222). A counter nut 381 is used to secure the threaded rod 288 (preventing axial displacement without concomitant rotational displacement thereof with respect to the frame 210 without hindering rotational movement necessary for rotating to and from the second orientation). When the knurled nut 288 is set to a position on the threaded rod 288 where the maximum force that can be exerted for the magnets used is reached, any further axial displacement of the threaded rod 288 enforced by the knurled nut 380 away from the second magnet 222 causes a) a reduction of the maximum force exerted by the clamp, and b) a reduction of friction when rotating the magnet 211 to the first orientation. The latter is the second advantage.

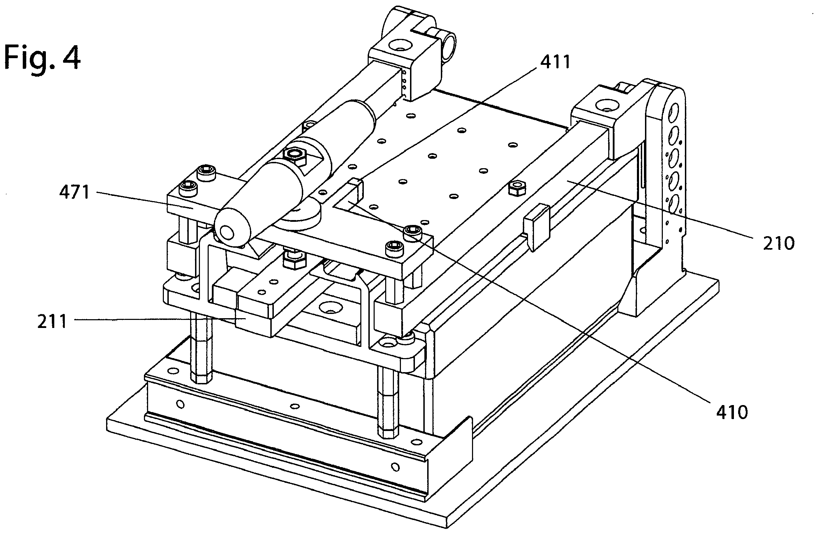

Fig. 4 corresponds to Fig. 2D, except that a cross-beam 471 part of frame 210 comprises a cantilever 410 having at its distal end a magnet 411 to bias the first magnet 211 to the first orientation when it is relatively close to said first orientation. This facilitates reliable gripping by a robot arm.

The invention may be varied within the scope of the appended claims. For example, instead of adjusting the height of the clamp, the height of the protruding element may be adjustable. The protruding element may also be hingedly attached to the frame, allowing it to be out of the way when the frame is in the upright position.

## Claims

1. A method of incubating cells in a cell-growth unit, wherein the cell-growth unit is placed on a moveable base plate of a shaker device, said shaker device comprising a clamp, said clamp comprising a frame, wherein the method of incubating the cells comprises the steps of
- clamping of the cell-growth unit to the base plate,
- performing the incubation of the cells, and
- releasing the clamp to release the cell-growth unit;
**characterized in that** a shaker device a shaker device is used, wherein
- the clamp is at a first end of said frame attached to the base plate, wherein the clamp is rotatable about a first axis of rotation parallel to the base plate, and at a second end of the frame opposite of the first end the frame is provided with a first oblong ferromagnetic element, wherein the frame is rotatable about the first axis from a first position in which the frame is in an upright orientation to a second position in which the second end of the frame is relatively close to the base plate,
- the base plate is provided with a second oblong ferromagnetic element,
with at least one of the first and the second ferromagnetic element Being a magnet,
wherein in the second position the first and second ferromagnetic element are relatively rotatable about a second axis of rotation from a first orientation in which the oblong first and second ferromagnetic elements are transverse to each other and are relatively not magnetically attracted to a second orientation in which said elements are aligned in their longitudinal directions and are relatively magnetically attracted, wherein the relative movement of one element chosen from the first and second ferromagnetic element from the first orientation to the second orientation defines a first plane and in the second orientation the longitudinal direction of the other element of the first and second ferromagnetic element is in a second plane parallel to said first plane,
wherein the step of clamping the cell-growth unit to the base plate comprises
- placing the cell-growth unit between the location where the first end of the frame is attached to the base plate and the location of the second oblong ferromagnetic element,
- moving the frame from the first position to the second position, and
- with the frame in the second position relatively moving the elements to the second orientation to sandwich the cell-growth unit between the frame and the base plate, and
wherein the step of releasing the clamp comprises relatively moving the elements towards the first orientation and then moving the frame to the first position.

2. The method according to claim 1, wherein, the frame clamps the growth-unit to the base plate via a protruding element facing the base plate in the second position at a location between 35-65% of the distance between a first end of the growth-unit closest to the first end of the frame and a second end of the growth-unit opposite of the first end of the growth unit.

3. The method according to claim 1 or 2, wherein the first and the second ferromagnetic element are magnets.

4. The method according to any of the preceding claims, the first oblong ferromagnetic element is rotatably mounted to the frame.

5. The method according to any of the preceding claims, wherein the first oblong ferromagnetic element is mounted to the frame using a threaded axle.

6. The method according to claim 5, wherein rotating the first ferromagnetic element from the second to the first orientation corresponds to an axial movement of the first ferromagnetic element of the second plane towards the first plane.

7. The method according to any of the preceding claims, wherein, the base plate is provided with a barrier that blocks the movement of the frame towards the second ferromagnetic element if the first ferromagnetic element is in the second orientation and allows the rotation from the first orientation to the second orientation of the first ferromagnetic element when said first ferromagnetic element has passed said barrier.

8. The method according to any of the preceding claims, wherein at least one of the base plate and the second end of the frame is provided with a stop ensuring an air gap between the oblong ferromagnetic elements when in the second orientation and second position.

9. The method according to any of the preceding claims, wherein the length to width ratios of the oblong ferromagnetic elements are independently chosen to be at least 3, and preferably at least 4.

10. The method according to any of the preceding claims, wherein the length to width ratios of the oblong ferromagnetic elements are independently chosen to be at most 10, and preferably at most 8.

11. The method according to any of the preceding claims, wherein the length to thickness ratio of the oblong ferromagnetic elements is preferably at least 10, and more preferably at least 14.

12. A clamp (100) for clamping a cell-growth unit to a base plate (181) of a shaker device (180), said clamp comprising a frame (210), said frame
- having a first end (201) for attachment to the base plate, wherein the clamp is rotatable about a first axis of rotation parallel to the base plate, and
- at a second end (202) of the frame opposite of the first end the frame being provided with a first oblong ferromagnetic element (211), wherein the frame is rotatable about the first axis from a first position in which the frame is in an upright orientation to a second position in which the second end of the frame is relatively close to the base plate,
wherein the clamp comprises a second oblong ferromagnetic element (222) for attachment to the base plate,
with at least one of the first and the second ferromagnetic element being a magnet,
wherein in the second position the first and second ferromagnetic element are relatively rotatable about a second axis of rotation from a first orientation in which the oblong first and second ferromagnetic elements are transverse to each other and are relatively not magnetically attracted to a second orientation in which said elements are aligned in their longitudinal directions and are relatively magnetically attracted, wherein the relative movement of one element chosen from the first and second ferromagnetic element from the first orientation to the second orientation defines a first plane and in the second orientation the longitudinal direction of the other element of the first and second ferromagnetic element is in a second plane parallel to said first plane.

## Patentansprüche

1. Verfahren zum Inkubieren von Zellen in einer Zellwachstumseinheit, wobei die Zellwachstumseinheit auf einer beweglichen Grundplatte einer Schüttelvorrichtung platziert ist, wobei die Schüttelvorrichtung eine Klemme umfasst, wobei die Klemme einen Rahmen umfasst, wobei das Verfahren zum Inkubieren der Zellen die Schritte umfasst von
- Festklemmen der Zellwachstumseinheit an der Grundplatte,
- Durchführen der Inkubation der Zellen und
- Lösen der Klemme, um die Zellwachstumseinheit freizugeben;
**dadurch gekennzeichnet, dass** eine Schüttelvorrichtung verwendet wird, wobei
- die Klemme an einem ersten Ende des Rahmens an der Grundplatte befestigt ist, wobei die Klemme um eine erste Drehachse parallel zur Grundplatte drehbar ist, und an einem zweiten Ende des Rahmens, das dem ersten Ende gegenüberliegt, der Rahmen mit einem ersten länglichen ferromagnetischen Element versehen ist, wobei der Rahmen um die erste Achse von einer ersten Position, in der sich der Rahmen in einer aufrechten Ausrichtung befindet, in eine zweite Position, in der sich das zweite Ende des Rahmens relativ nahe an der Grundplatte befindet, drehbar ist,
- die Grundplatte mit einem zweiten länglichen ferromagnetischen Element versehen ist, wobei mindestens eines von dem ersten und dem zweiten ferromagnetischen Element ein Magnet ist,
wobei in der zweiten Position das erste und das zweite ferromagnetische Element relativ um eine zweite Drehachse von einer ersten Ausrichtung, in der das längliche erste und zweite ferromagnetische Element quer zueinander stehen und relativ nicht magnetisch angezogen werden, in eine zweite Ausrichtung, in der die Elemente in ihren Längsrichtungen ausgerichtet sind und relativ magnetisch angezogen werden, drehbar sind, wobei die relative Bewegung eines Elements, das aus dem ersten und zweiten ferromagnetischen Element ausgewählt ist, von der ersten Ausrichtung in die zweite Ausrichtung eine erste Ebene definiert und in der zweiten Ausrichtung die Längsrichtung des anderen Elements des ersten und zweiten ferromagnetischen Elements in einer zweiten Ebene parallel zur ersten Ebene liegt,
wobei der Schritt des Festklemmens der Zellwachstumseinheit an der Grundplatte umfasst
- Platzieren der Zellwachstumseinheit zwischen der Stelle, an der das erste Ende des Rahmens an der Grundplatte befestigt ist, und der Stelle des zweiten länglichen ferromagnetischen Elements,
- Bewegen des Rahmens von der ersten Position in die zweite Position, und
- wobei der Rahmen in der zweiten Position die Elemente relativ in die zweite Ausrichtung bewegt, um die Zellwachstumseinheit zwischen dem Rahmen und der Grundplatte einzuklemmen, und
wobei der Schritt des Lösens der Klemme das relative Bewegen der Elemente in Richtung der ersten Ausrichtung und dann das Bewegen des Rahmens in die erste Position umfasst.

2. Verfahren nach Anspruch 1, wobei der Rahmen die Wachstumseinheit über ein vorstehendes Element, das der Grundplatte in der zweiten Position zugewandt ist, an einer Stelle zwischen 35-65% des Abstands zwischen einem ersten Ende der Wachstumseinheit, das dem ersten Ende des Rahmens am nächsten liegt, und einem zweiten Ende der Wachstumseinheit, das dem ersten Ende der Wachstumseinheit gegenüberliegt, an der Grundplatte festklemmt.

3. Verfahren nach Anspruch 1 oder 2, wobei das erste und das zweite ferromagnetische Element Magnete sind.

4. Verfahren nach einem der vorhergehenden Ansprüche, wobei das erste längliche ferromagnetische Element drehbar am Rahmen montiert ist.

5. Verfahren nach einem der vorhergehenden Ansprüche, wobei das erste längliche ferromagnetische Element unter Verwendung einer Gewindeachse am Rahmen montiert ist.

6. Verfahren nach Anspruch 5, wobei das Drehen des ersten ferromagnetischen Elements von der zweiten in die erste Ausrichtung einer axialen Bewegung des ersten ferromagnetischen Elements von der zweiten Ebene in Richtung der ersten Ebene entspricht.

7. Verfahren nach einem der vorhergehenden Ansprüche, wobei die Grundplatte mit einer Barriere versehen ist, die die Bewegung des Rahmens in Richtung des zweiten ferromagnetischen Elements blockiert, wenn sich das erste ferromagnetische Element in der zweiten Ausrichtung befindet, und die Drehung von der ersten Ausrichtung in die zweite Ausrichtung des ersten ferromagnetischen Elements ermöglicht, wenn das erste ferromagnetische Element die Barriere passiert hat.

8. Verfahren nach einem der vorhergehenden Ansprüche, wobei mindestens eines von der Grundplatte und dem zweiten Ende des Rahmens mit einem Anschlag versehen ist, der einen Luftspalt zwischen den länglichen ferromagnetischen Elementen in der zweiten Ausrichtung und zweiten Position gewährleistet.

9. Verfahren nach einem der vorhergehenden Ansprüche, wobei die Längen-zu-Breiten-Verhältnisse der länglichen ferromagnetischen Elemente unabhängig voneinander so gewählt werden, dass sie mindestens 3 und vorzugsweise mindestens 4 betragen.

10. Verfahren nach einem der vorhergehenden Ansprüche, wobei die Längen-zu-Breiten-Verhältnisse der länglichen ferromagnetischen Elemente unabhängig voneinander so gewählt werden, dass sie höchstens 10 und vorzugsweise höchstens 8 betragen.

11. Verfahren nach einem der vorhergehenden Ansprüche, wobei das Längen-zu-Breiten-Verhältnis der länglichen ferromagnetischen Elemente vorzugsweise mindestens 10 und noch bevorzugter mindestens 14 beträgt.

12. Klemme (100) zum Festklemmen einer Zellwachstumseinheit an einer Grundplatte (181) einer Schüttelvorrichtung (180), wobei die Klemme einen Rahmen (210) umfasst, wobei der Rahmen
- ein erstes Ende (201) zur Befestigung an der Grundplatte aufweist, wobei die Klemme um eine erste Drehachse parallel zur Grundplatte drehbar ist, und
- an einem zweiten Ende (202) des Rahmens, das dem ersten Ende des Rahmens gegenüberliegt, der Rahmen mit einem ersten länglichen ferromagnetischen Element (211) versehen ist, wobei der Rahmen um die erste Achse von einer ersten Position, in der sich der Rahmen in einer aufrechten Ausrichtung befindet, in eine zweite Position, in der sich das zweite Ende des Rahmens relativ nahe an der Grundplatte befindet, drehbar ist,
wobei die Klemme ein zweites längliches ferromagnetisches Element (222) zur Befestigung an der Grundplatte umfasst,
wobei mindestens eines von dem ersten und dem zweiten ferromagnetischen Element ein Magnet ist, wobei in der zweiten Position das erste und das zweite ferromagnetische Element relativ um eine zweite Drehachse von einer ersten Ausrichtung, in der das längliche erste und zweite ferromagnetische Element quer zueinander stehen und relativ nicht magnetisch angezogen werden, in eine zweite Ausrichtung, in der die Elemente in ihren Längsrichtungen ausgerichtet sind und relativ magnetisch angezogen werden, drehbar sind, wobei die relative Bewegung eines Elements, das aus dem ersten und zweiten ferromagnetischen Element ausgewählt ist, von der ersten Ausrichtung in die zweite Ausrichtung eine erste Ebene definiert und in der zweiten Ausrichtung die Längsrichtung des anderen Elements des ersten und zweiten ferromagnetischen Elements in einer zweiten Ebene parallel zur ersten Ebene liegt.

## Revendications

1. Procédé d'incubation de cellules dans une unité de croissance cellulaire, ladite unité de croissance cellulaire étant placée sur une plaque de base mobile d'un dispositif secoueur, ledit dispositif secoueur comprenant une pince, ladite pince comprenant un cadre, ledit procédé d'incubation des cellules comprenant les étapes de
- serrage de l'unité de croissance cellulaire sur la plaque de base,
- réalisation de l'incubation des cellules, et
- relâchement de la pince pour libérer l'unité de croissance cellulaire ;
**caractérisé par le fait que**
un dispositif secoueur est utilisé,
- ladite pince étant fixée au niveau d'une première extrémité dudit cadre à la plaque de base, ladite pince pouvant tourner autour d'un premier axe de rotation parallèle à la plaque de base, et au niveau d'une seconde extrémité du cadre opposée à la première extrémité, ledit cadre étant doté d'un premier élément ferromagnétique oblong, ledit cadre pouvant tourner autour du premier axe d'une première position dans laquelle le cadre se trouve dans une orientation verticale à une seconde position dans laquelle la seconde extrémité du cadre est relativement proche de la plaque de base,
- ladite plaque de base étant dotée d'un second élément ferromagnétique oblong, au moins un élément parmi le premier et le second élément ferromagnétique étant un aimant,
dans la seconde position ledit premier et ledit second élément ferromagnétique pouvant tourner de manière relative autour d'un second axe de rotation d'une première orientation dans laquelle les premier et second éléments ferromagnétiques oblongs sont transversaux l'un par rapport à l'autre et ne sont relativement pas attirés magnétiquement vers une seconde orientation dans laquelle lesdits éléments sont alignés dans leurs sens longitudinaux et sont relativement attirés magnétiquement, le déplacement relatif d'un élément choisi parmi le premier et le second élément ferromagnétique de la première orientation à la seconde orientation définissant un premier plan et dans la seconde orientation ledit sens longitudinal de l'autre élément parmi le premier et le second élément ferromagnétique se trouvant dans un second plan parallèle audit premier plan, ladite étape de serrage de l'unité de croissance cellulaire sur la plaque de base comprenant
- le placement de l'unité de croissance cellulaire entre l'emplacement dans lequel la première extrémité du cadre est fixée à la plaque de base et l'emplacement du second élément ferromagnétique oblong,
- le déplacement du cadre de la première position à la seconde position, et
- le cadre se trouvant dans la seconde position, le déplacement relatif des éléments vers la seconde orientation pour prendre en sandwich l'unité de croissance cellulaire entre le cadre et la plaque de base, et
ladite étape de relâchement de la pince comprenant le déplacement relatif des éléments en direction de la première orientation, puis le déplacement du cadre vers la première position.

2. Procédé selon la revendication 1, ledit cadre serrant l'unité de croissance sur la plaque de base par le biais d'un élément saillant faisant face à la plaque de base dans la seconde position au niveau d'un emplacement compris entre 35 à 65 % de la distance entre une première extrémité de l'unité de croissance la plus proche de la première extrémité du cadre et une seconde extrémité de l'unité de croissance opposée à la première extrémité de l'unité de croissance.

3. Procédé selon la revendication 1 ou 2, ledit premier et ledit second élément ferromagnétique étant des aimants.

4. Procédé selon l'une quelconque des revendications précédentes, ledit premier élément ferromagnétique oblong étant monté rotatif sur le cadre.

5. Procédé selon l'une quelconque des revendications précédentes, ledit premier élément ferromagnétique oblong étant monté sur le cadre à l'aide d'un axe fileté.

6. Procédé selon la revendication 5, ladite rotation du premier élément ferromagnétique de la seconde à la première orientation correspondant à un déplacement axial du premier élément ferromagnétique du second plan en direction du premier plan.

7. Procédé selon l'une quelconque des revendications précédentes, ladite plaque de base étant dotée d'une barrière qui bloque le déplacement du cadre en direction du second élément ferromagnétique si le premier élément ferromagnétique se trouve dans la seconde orientation et permet la rotation de la première orientation à la seconde orientation du premier élément ferromagnétique lorsque ledit premier élément ferromagnétique a franchi ladite barrière.

8. Procédé selon l'une quelconque des revendications précédentes, au moins une parmi la plaque de base et la seconde extrémité du cadre étant muni d'une butée assurant un entrefer entre les éléments ferromagnétiques oblongs lorsqu'ils se trouvent dans la seconde orientation et la seconde position.

9. Procédé selon l'une quelconque des revendications précédentes, les rapports longueur sur largeur des éléments ferromagnétiques oblongs étant indépendamment choisis de manière à être supérieurs ou égaux à 3, et de préférence supérieurs ou égaux à 4.

10. Procédé selon l'une quelconque des revendications précédentes, lesdits rapports longueur sur largeur des éléments ferromagnétiques oblongs étant indépendamment choisis de manière à être inférieurs ou égaux à 10, et de préférence inférieurs ou égaux à 8.

11. Procédé selon l'une quelconque des revendications précédentes, ledit rapport longueur sur épaisseur des éléments ferromagnétiques oblongs étant de préférence supérieur ou égal à 10, et plus préférablement supérieur ou égal à 14.

12. Pince (100) destinée à serrer une unité de croissance cellulaire sur une plaque de base (181) d'un dispositif secoueur (180), ladite pince comprenant un cadre (210), ledit cadre
- comportant une première extrémité (201) destinée à la fixation sur la plaque de base, ladite pince pouvant tourner autour d'un premier axe de rotation parallèle à la plaque de base, et
- au niveau d'une seconde extrémité (202) du cadre opposée à la première extrémité, le cadre étant doté d'un premier élément ferromagnétique oblong (211), ledit cadre pouvant tourner autour du premier axe d'une première position dans laquelle le cadre se trouve dans une orientation verticale à une seconde position dans laquelle la seconde extrémité du cadre est relativement proche de la plaque de base,
ladite pince comprenant un second élément ferromagnétique oblong (222) permettant la fixation sur la plaque de base,
au moins l'un des premier et second éléments ferromagnétiques étant un aimant, dans la seconde position lesdits premier et second éléments ferromagnétiques pouvant tourner de manière relative autour d'un second axe de rotation d'une première orientation dans laquelle les premier et second éléments ferromagnétiques oblongs sont transversaux l'un par rapport à l'autre et ne sont relativement pas attirés magnétiquement vers une seconde orientation dans laquelle lesdits éléments sont alignés dans leurs sens longitudinaux et sont relativement attirés magnétiquement, ledit mouvement relatif d'un élément choisi parmi le premier et le second élément ferromagnétique de la première orientation à la seconde orientation définissant un premier plan et dans la seconde orientation le sens longitudinal de l'autre élément parmi le premier et le second élément ferromagnétique se trouvant dans un second plan parallèle audit premier plan.
